# EUROPEAN PATENT SPECIFICATION

(11) **EP 3 547 955 B1**
(45) Date of publication and mention of the grant of the patent: **24.03.2021**
(21) Application number: 17808890.2
(22) Date of filing: 01.12.2017
(51) Int. Cl.: A61C 17/16, A61C 17/22

(54) **METHOD AND SYSTEM FOR CALIBRATING AN ORAL CLEANING DEVICE**
VERFAHREN UND SYSTEM ZUR KALIBRIERUNG EINER MUNDREINIGUNGSVORRICHTUNG
PROCÉDÉ ET SYSTÈME POUR L'ÉTALONNAGE D'UN DISPOSITIF DE NETTOYAGE BUCCAL

(30) Priority: 01.12.2016 US 201662428735 P
(43) Date of publication of application: 09.10.2019
(73) Proprietor: Koninklijke Philips N.V., 5656 AG Eindhoven (NL)
(72) Inventor: JEANNE, Vincent, 5656 AE Eindhoven (NL); HARDEMAN, Toon, 5656 AE Eindhoven (NL); DEN HAMER, Abram, Jan, 5656 AE Eindhoven (NL)
(74) Representative: Schudelaro, Antonius Adrianus Petrus
(86) International application number: PCT/EP2017/081276
(87) International publication number: WO 2018/100198

(56) References cited:
- WO-A1-2015/140340
- WO-A1-2016/180929
- CN-A- 105 029 891

## Description

### Field of the Invention

The present disclosure is directed generally to methods and systems for calibrating a gyroscope of an oral cleaning device.

### Background

Proper oral care techniques, including length and coverage of cleaning, helps ensure long-term dental health. Many dental problems are experienced by individuals who either do not regularly clean their teeth or who do so inadequately. Among individuals who do regularly clean, improper cleaning habits can result in poor coverage of cleaning and thus surfaces that are not adequately cleaned.

To facilitate proper cleaning technique, it is important to ensure that there is adequate cleaning of all dental surfaces, including areas of the mouth that are hard to reach or that tend to be improperly cleaned during an average cleaning session. One way to ensure adequate coverage and cleaning is to track the movement and location of the oral cleaning device during cleaning. Typically, the oral cleaning device will comprise one or more sensors, such as a gyroscope among others, to facilitate localization and tracking.

However, sensors such as gyroscopes can exhibit a dependency on temperature that can introduce drift into the obtained sensor data, and can significantly affect the accuracy of localization and tracking estimates. To overcome this dependency on temperature, regular sensor calibration is necessary. This calibration is typically done either by the user, or automatically by the system. For example, during calibration the device should be in stable position such that the measured bias represents the zero rate condition of the gyroscope. Existing calibration methods, however, introduce a risk of not being able to reduce the impact of drift on calculations, as there is no guarantee that the system can properly characterize the temperature dependency.

Accordingly, there is a continued need in the art for methods and systems for accurate calibration of a gyroscope of an oral cleaning device.

WO 2015/140340 discloses an oral cleaning device with a first motion identifier for generating information indicative of the acceleration and/or angular orientation of the cleaning head. A second motion identifier generates information indicative of the acceleration and/or angular orientation of a reference member fixed to the body. Information indicative of the trajectory and/or orientation of the cleaning head relative to the part of the body to be treated is based on the information provided by the first and second motion identifiers.

WO 2016/180929 discloses a tooth brushing monitoring system comprising a toothbrush with sensors and a base station. The sensors record data regarding the quality, quantity and location of brushing and the system can analyze the data to provide feedback on the quality of brushing.

### Summary of the Invention

The invention is defined by the claims. The present disclosure is directed to inventive methods and systems for calibrating a gyroscope of an oral cleaning device. Applied to an oral cleaning device, the inventive methods and systems enable improved calibration of the gyroscope and thus allow for improved motion identification, tracking, and/or localization. The system obtains gyroscope sensor data for a plurality of time points, preferably at two or more different temperatures, and uses the data to generate a model of gyroscope response or behavior. The system then utilizes this calibration model to calibrate new gyroscope sensor data. According to an embodiment, the system tests the calibration model at a known temperature, comparing the output of gyroscope sensor data calibrated using the calibration model to actual data without calibration. According to a further embodiment, the user is advised to perform a calibration it the calibration model is determined to be invalid.

Generally in one aspect, a method for calibrating a gyroscope of an oral care device is provided. The method includes the steps of: (i) scheduling a data acquisition scheme comprising a plurality of time points; (ii) obtaining sensor data by the gyroscope at each of the plurality of time points; (iii) determining whether the oral care device was experiencing motion at any of the plurality of time points and, if so, discarding those time points from further analysis; (iv) determining a calibration model using the obtained sensor data; (v) generating, based on the obtained sensor data and the determined calibration model, one or more parameters describing a behavior of the gyroscope; and (vi) calibrating, using the one or more parameters, gyroscope data obtained during a cleaning session.

According to an embodiment, the calibration model comprises a calibration offset.

According to an embodiment, the data acquisition scheme is based at least in part on observed temperature data. According to an embodiment, the data acquisition scheme is based at least in part on one or more expected temperatures. According to an embodiment, the data acquisition scheme is designed maximize a range of temperatures for the plurality of time points, and minimize a chance of physical motion at the plurality of time points.

According to an embodiment, the method further includes the step of testing the determined calibration model at a known temperature.

According to an embodiment, the method further includes the step of requesting, if the determined calibration model is inaccurate, a calibration by a user of the oral care device.

According to an aspect is an oral care device configured to calibrate a sensor. The device includes: a gyroscope configured to obtain gyroscope data; and a controller configured to: (i) schedule a data acquisition scheme comprising a plurality of time points; (ii) receive sensor data from the gyroscope for each of the plurality of time points; (iii) determine whether the oral care device was experiencing motion at any of the plurality of time points and, if so, discarding those time points from further analysis; (iv) determine a calibration model using the obtained sensor data; (v) generate, based on the obtained sensor data and the determined calibration model, one or more parameters describing a behavior of the gyroscope; and (vi) calibrate, using the one or more parameters, gyroscope data obtained during a cleaning session.

According to an aspect is a cleaning device configured to calibrate a gyroscope. The device includes: (i) a scheduling module configured to schedule a data acquisition scheme comprising a plurality of time points; (ii) an acquisition module configured to obtain sensor data from the gyroscope for each of the plurality of time points, and further configured to determine whether the oral care device was experiencing motion at any of the plurality of time points and, if so, to discard those time points from further analysis; (iii) a model selection module configured to determine a calibration model using the obtained sensor data; (iv) a model calibration module configured to generate, based on the obtained sensor data and the determined calibration model, one or more parameters describing a behavior of the gyroscope; and (v) a model compensation module configured to calibrate, using the one or more parameters, gyroscope data obtained during a cleaning session.

According to an embodiment, the device further includes a model validation module configured to test the determined calibration model at a known temperature.

According to an embodiment, the device further includes a connected temperature module configured to provide temperature data to the scheduling module or the model compensation module.

As used herein for purposes of the present disclosure, the term "controller" is used generally to describe various apparatus relating to the operation of a stream probe apparatus, system, or method. A controller can be implemented in numerous ways (e.g., such as with dedicated hardware) to perform various functions discussed herein. A "processor" is one example of a controller which employs one or more microprocessors that may be programmed using software (e.g., microcode) to perform various functions discussed herein. A controller may be implemented with or without employing a processor, and also may be implemented as a combination of dedicated hardware to perform some functions and a processor (e.g., one or more programmed microprocessors and associated circuitry) to perform other functions. Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media (generically referred to herein as "memory," e.g., volatile and non-volatile computer memory). In some implementations, the storage media may be encoded with one or more programs that, when executed on one or more processors and/or controllers, perform at least some of the functions discussed herein. Various storage media may be fixed within a processor or controller or may be transportable, such that the one or more programs stored thereon can be loaded into a processor or controller so as to implement various aspects of the present disclosure discussed herein. The terms "program" or "computer program" are used herein in a generic sense to refer to any type of computer code (e.g., software or microcode) that can be employed to program one or more processors or controllers.

The term "user interface" as used herein refers to an interface between a human user or operator and one or more devices that enables communication between the user and the device(s). Examples of user interfaces that may be employed in various implementations of the present disclosure include, but are not limited to, switches, potentiometers, buttons, dials, sliders, track balls, display screens, various types of graphical user interfaces (GUIs), touch screens, microphones and other types of sensors that may receive some form of human-generated stimulus and generate a signal in response thereto.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the invention will be apparent from and elucidated with reference to the embodiment(s) described hereinafter.

### Brief Description of the Drawings

In the drawings, like reference characters generally refer to the same parts throughout the different views. Also, the drawings are not necessarily to scale, emphasis instead generally being placed upon illustrating the principles of the invention.
FIG. 1 is a representation of an oral cleaning device in accordance with an embodiment.
FIG. 2 is a schematic representation of an oral cleaning system in accordance with an embodiment.
FIG. 3 is a schematic representation of an oral cleaning system in accordance with an embodiment.
FIG. 4 is a flowchart of a method for calibrating the gyroscope of an oral cleaning device in accordance with an embodiment.

### Detailed Description of Embodiments

The present disclosure describes various embodiments of a method and system for calibrating a gyroscope of an oral cleaning device. More generally, Applicant has recognized and appreciated that it would be beneficial to provide a system that calibrates a gyroscope for a range of different temperatures, understanding that gyroscope data can be temperature sensitive. Accordingly, the methods and systems described or otherwise envisioned herein provide an oral cleaning device configured to obtain gyroscope sensor data for a plurality of time points at different temperatures, and to use that data to generate a model of gyroscope behavior. The oral cleaning device then utilizes this calibration model to calibrate new gyroscope sensor data. According to an embodiment, the oral cleaning device tests the calibration model at a known temperature, comparing the output of gyroscope sensor data calibrated using the calibration model to actual data without calibration. If the calibration model is determined to be invalid, the user may be advised to perform a calibration.

The embodiments and implementations disclosed or otherwise envisioned herein can be utilized with any oral care device. Examples of suitable personal care devices include a toothbrush, a flossing device, an oral irrigator, a tongue cleaner, or other oral care device. However, the disclosure is not limited to these enumerated devices, and thus the disclosure and embodiments disclosed herein can encompass any oral care device.

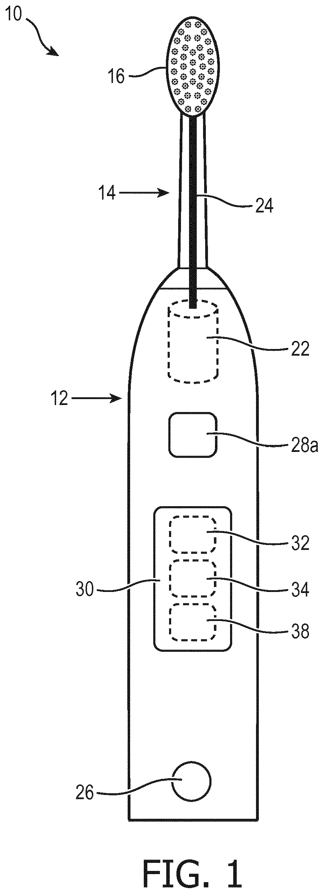

Referring to FIG. 1, in one embodiment, an oral care device 10 is provided that includes a handle or body portion 12 and a head member 14. Head member 14 includes at its end remote from the body portion a head 16. The body portion 12 typically comprises a housing, at least a portion of which is hollow, to contain components of the personal care device. According to an embodiment, head member 14 is mounted so as to be able to move relative to the body portion 12. The movement can be any of a variety of different movements, including vibrations or rotation, among others. Although in the present embodiment the oral care device 10 is an oscillating toothbrush, it will be understood that alternative embodiment the oral care devices are also within the scope of the present invention.

The body portion 12 can comprise a drivetrain assembly with a motor 22 for generating movement, and a transmission component or drivetrain shaft 24, for transmitting the generated movements to head member 14. For example, the drivetrain comprises a motor or electromagnet(s) 22 that generates movement of a drivetrain shaft 24, which is subsequently transmitted to the head member 14. The drivetrain can include components such as a power supply, an oscillator, and one or more electromagnets, among other components. In this embodiment the power supply comprises one or more rechargeable batteries, not shown, which can, for example, be electrically charged in a charging holder in which oral care device 10 is placed when not in use. According to one embodiment, head member 14 is mounted to the drive train shaft 24 so as to be able to move relative to body portion 12. The head member 14 can be fixedly mounted onto drive train shaft 24, or it may alternatively be detachably mounted so that head member 14 can be replaced with a different head member for different operating features, or when a component of the head are worn out and require replacement. Body portion 12 is further provided with a user input 26 to activate and de-activate the drivetrain. The user input 26 allows a user to operate the oral care device 10, for example to turn the device on and off. The user input 26 may, for example, be a button, touch screen, or switch.

Oral care device 10 includes one or more sensors 28, one of which is a gyroscope. Gyroscope 28a is shown in FIG. 1 within body portion 12, but may be located anywhere within the device, including for example within head member 14. In addition to a gyroscope, other sensors may be present in the oral care device. According to an embodiment, sensors 28 are configured to provide readings of six axes of relative motion (three axes translation and three axes rotation), using for example a 3-axis gyroscope 28a and a 3-axis accelerometer. As another example, sensors 28 are configured to provide the readings of nine axes of relative motion using, for example, 3-axis gyroscope 28a, a 3-axis accelerometer, and a 3-axis magnetometer. Other possible types of sensors can include, but are not limited to a pressure sensor, a capacitive sensor, a camera, a photocell, a clock, a timer, and other types of devices. Many different types of sensors could be utilized, as described or otherwise envisioned herein. According to an embodiment, sensors 28 are configured to generate information indicative of the acceleration and angular orientation of oral care device 10.

Data generated by gyroscope 28a is provided to a controller 30. According to an embodiment, the gyroscope 28a could be integral to controller 30. Controller 30 may be formed of one or multiple modules, and is configured to operate the personal care device 10 in response to an input, such as input obtained via user input 26. Controller 30 can comprise, for example, a processor 32 and a memory 34. Processor 32 may take any suitable form, including but not limited to a microcontroller, multiple microcontrollers, circuitry, a single processor, or plural processors. Memory 34 can take any suitable form, including a non-volatile memory and/or RAM. The non-volatile memory may include read only memory (ROM), a hard disk drive (HDD), or a solid state drive (SSD). The memory can store, among other things, an operating system. The RAM is used by the processor for the temporary storage of data. According to an embodiment, an operating system may contain code which, when executed by controller 30, controls operation of the hardware components of oral care device 10. According to an embodiment, connectivity module 38 transmits collected sensor data, and can be any module, device, or means capable of transmitting a wired or wireless signal, including but not limited to a Wi-Fi, Bluetooth, near field communication, and/or cellular module.

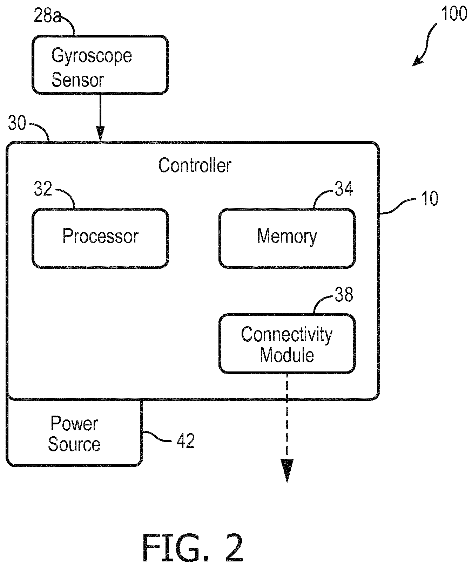

Referring to FIG. 2, in one embodiment, a schematic representation of the control system 100 of oral care device 10 is provided. The control system 100 of oral care device 10 comprise a controller 30 with a processor 32 and a memory 34, which can store an operating system as well as sensor data. The device also comprises a power source 42 which can be AC power, or can be battery power from a rechargeable battery. The control system 100 further comprises a gyroscope sensor 28a. In addition to the gyroscope, the device may comprise other sensors. The one or more sensors 28 generate sensor data and communicate that data to controller 30. The control system 100 further comprises a connectivity module 38 configured and/or programmed to transmit sensor data to a wireless transceiver (not shown). For example, connectivity module 38 may transmit sensor data via a Wi-Fi connection over the Internet or an Intranet to a dental professional, a database, or other location. Alternatively, connectivity module 38 may transmit sensor or feedback data via a Bluetooth or other wireless connection to a local device (e.g., a separate computing device), database, or other transceiver. For example, connectivity module 38 allows the user to transmit sensor data to a separate database to be saved for long-term storage, to transmit sensor data for further analysis, to transmit user feedback to a separate user interface, or to share data with a dental professional, among other uses.

According to an embodiment, control system 100 of oral care device 10 can programmed and/or configured to calibrate the gyroscope 28a for a range of different temperatures and/or over a course of time. As discussed herein, the information or data analyzed or used by control system 100 of oral care device 10 to carry out the functions and methods described herein can be generated by gyroscope sensor 28a. Accordingly, control system 100 receives gyroscope sensor data for a plurality of time points at different temperatures and/or different times, and uses that data to generate a model of gyroscope behavior. The control system can also test the calibration model at one or more known temperatures, and can request that the user perform a calibration if necessary.

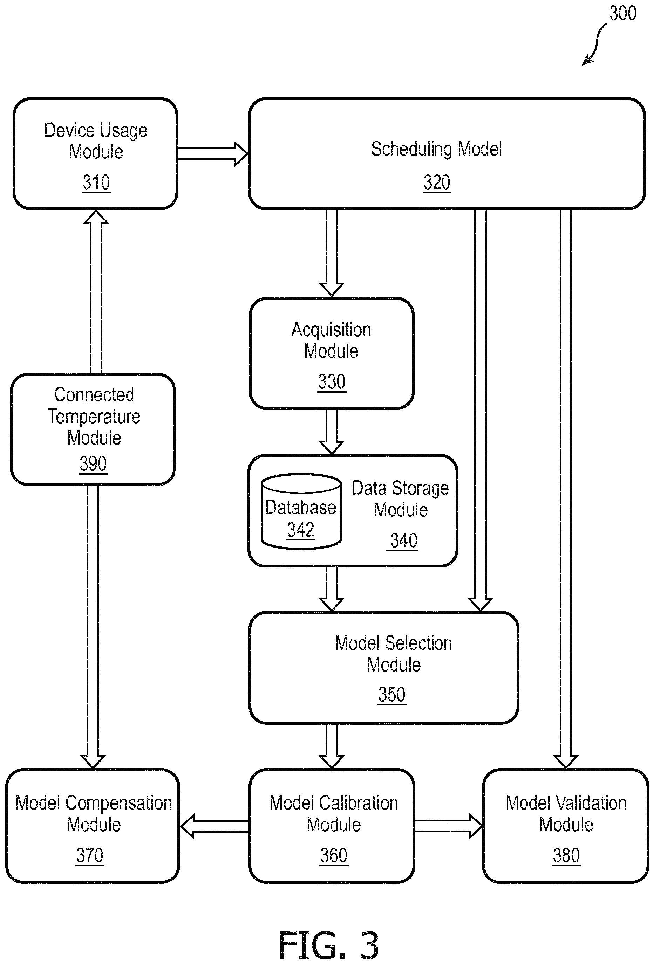

Referring to FIG. 3, in one embodiment, is an oral cleaning system 300. Oral cleaning system 300 is an embodiment of oral cleaning device 10, which can be any of the oral cleaning device embodiments disclosed or otherwise envisioned herein. According to another embodiment, oral cleaning system 300 can be implemented in two or more devices. For example, one or more of the modules or components of oral cleaning system 300 can be implemented in a remote device such as a smartphone, tablet, wearable device, or other computer.

According to an embodiment, oral cleaning system 300 comprises a device usage module 310. The device usage module registers information about what time and for what duration the oral cleaning device, with gyroscope 28a, is used. This information is typically stored for future analysis and processing. The type of use could also be registered, such as whether the use was a charging event or a cleaning event. This information can be obtained directly from the oral cleaning device itself.

The oral cleaning system 300 further comprises a scheduling module 320. The scheduling module generates a data acquisition scheme based on typical device usage, which can be based on historical device usage and/or on average device usage derived from experimental or surveyed data. According to an embodiment, the data acquisition scheme is designed to both: (i) maximize the temperature range between the data points; and (ii) minimize the chance of physical motion during calibration. For example, the scheduling module can select calibration data to be obtained while charging, and shortly after a cleaning session. This maximizes temperature spread due to the heating up of a device during use, and minimizes the chance that the device will be moving during the calibration.

According to an embodiment, scheduling module 320 of oral cleaning system 300 is further programmed, configured, or designed to select among calibration models and perform calibration before each actual use of the oral cleaning device. The scheduling module 320 also triggers a model validation routine, described below, to check the validity of the selected model for the actual temperature. If the model is determined to be invalid, scheduling module 320 can trigger the device to acquire a new data point and inform the user to perform a calibration procedure, such as putting it on a stable flat surface.

The oral cleaning system 300 further comprises an acquisition module 330 programmed or configured to collect data at one or more time points and/or over a certain time period, as defined by the scheduling module 320. The acquisition module 330 also determines whether the oral care device was experience motion at any one of the plurality of scheduled time points. If motion is detected, the collected data is discarded. According to an embodiment, a new data acquisition scheme may be scheduled, or the existing data may be utilized after one or more time points are discarded. The motion could be based on gyroscope data, but could also utilize other sensors such as an accelerometer or other indicators such as an active motor. For every valid data trace, the mean value is computed and stored.

Accordingly, the oral cleaning system 300 further comprises a data storage module 340 configured or programmed to store gyroscope data, estimated gyroscope offsets and corresponding temperature values, and/or other data in a database, such as database 342 which can be any of the memory or databases described or otherwise envisioned herein. According to an embodiment, the database stores the last 1,000 calibration points. According to another embodiment, the database stores only calibration points that result in a large temperature spread.

The oral cleaning system 300 further comprises a model selection module 350 configured or programmed to select a calibration model based on: (i) the data stored by data storage module 340; (ii) prior knowledge about the expected model; and (iii) a requested accuracy, which can be determined by a user or preprogrammed or predetermined by manufacturer settings. According to an embodiment, selecting a model comprises a trade-off between a low deterministic or stochastic error. Complex models may result in small deterministic errors, but if the amount of data points is low then stochastic error could be quite large. According to an embodiment, therefore, the complexity of the model is adapted to the available data. Typically, initially only a calibration offset will be estimated. If sufficient data is present over a sufficiently large temperature range, a higher order polynomial calibration model can be estimated.

The oral cleaning system 300 further comprises a model calibration module 360 configured or programmed to generate one or more parameters which describe the behavior of the oral care device, specifically the gyroscope, based on: (i) the data stored by the data storage module 340; and (ii) the calibration model selected by the model selection module 350. Commonly used techniques like least squares estimates can be utilized for the generation of parameters.

According to an embodiment, the model calibration module 360 generates one or more parameters before each cleaning session, assuming that new calibration data is available. However, if there is currently a valid calibration model, the parameters can be generated during and/or after use as well. According to an embodiment, it is possible to link the model calibration module 360 to the acquisition module 330 such that a new model is fitted whenever there is a new data point present.

The oral cleaning system 300 further comprises a model compensation module 370 configured or programmed to calibrate, using the selected model and the one or more parameters, gyroscope data obtained during a cleaning session. According to an embodiment, the model compensation module 370 uses the selected model and one or more parameters to correct for the offset in the measured gyroscope signals.

The oral cleaning system 300 further comprises a model validation module 380 configured or programmed to validate the selected model. According to an embodiment, the validity of the select model at a given condition, such as a known temperature, can be determined using the selected model, the one or more parameters, and the stored data points. According to another embodiment, the given condition could be, for example, a time course. Similarly, the one or more parameters may comprise a time or time course aspect.

Optionally, the oral cleaning system 300 further comprises a connected temperature module 390 configured or programmed to provide one or more temperature inputs to the system. For example, calibration sample acquisition is triggered by an external measurement device, such as a connected internal and/or external thermometer. Calibration sample acquisition may be triggered, for example, if a temperature not previously observed, meaning no calibration samples are available, is detected by the connected temperature module. The system can then autonomously collect calibration samples without requiring use of the oral device. According to another embodiment, by exploiting the daily program of the central heating system, the optimal data acquisition moments for the scheduling module could be determined to maximize temperature variation as described herein.

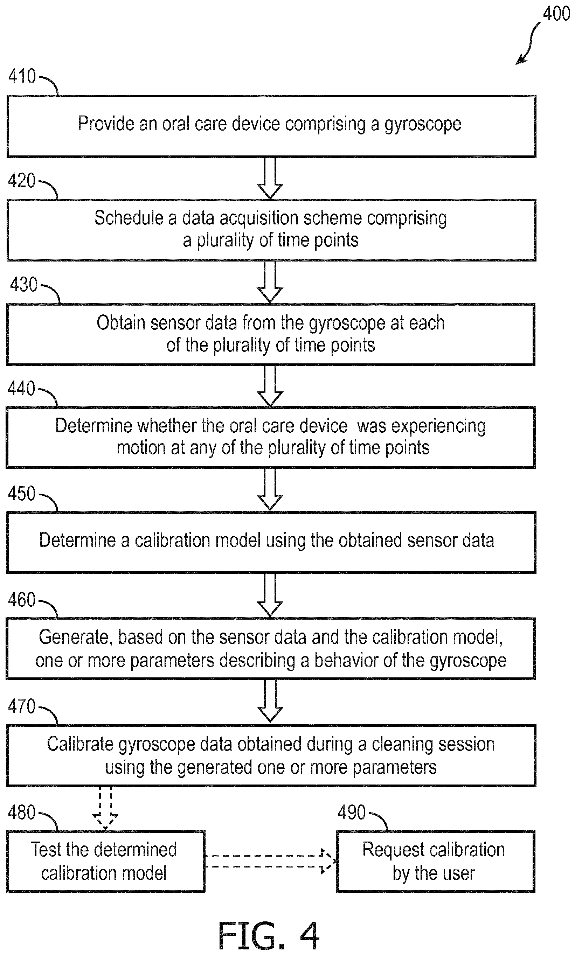

Referring to FIG. 4, in one embodiment, is a flowchart of a method 400 for calibrating a gyroscope of an oral care device. In step 410 of the method, an oral care device 10 is provided. Oral care device 10 can be any of the devices described or otherwise envisioned herein.

At step 420 of the method, a data acquisition scheme comprising a plurality of time points is scheduled. For example, the data acquisition scheme can be based on typical device usage, which can be based on historical device usage and/or on average device usage derived from experimental or surveyed data. According to an embodiment, the data acquisition scheme is designed to both: (i) maximize the temperature range between the data points; and (ii) minimize the chance of physical motion during calibration. For example, the schedule can be designed to obtained calibration data while charging, and shortly after a cleaning session. The schedule can also be determined in part by an actual temperature from an internal and/or external temperature sensor, such as thermometer. The schedule can also be determined in part by an expected temperature, for example from a connected heating system, among other embodiments.

At step 430 of the method, the device obtains sensor data from the gyroscope 28a at each of the plurality of time points set forth in the data acquisition scheme. According to an embodiment, the data is obtained at two or more different temperatures. The obtained data can then be stored for future or aggregate analysis.

According to an embodiment, the collected data comprises and/or is associated with a time stamp or other element configured to identify the collected data based on the time and/or date it was collected, or to identify the collected data by age. For example, since sensors can drift over time, it could be beneficial to emphasize new data and discard old calibration data. During model estimation, therefore, the system could apply a time weighting based on a time stamp at which the data was collected. For example, the system could discard calibration data that is a certain amount of time old, and/or could weight new data higher than older data. This will help minimize the effect of sensor drift on calibration.

At step 440 of the method, the oral care device analyzes the sensor data from the gyroscope for the plurality of time points set forth in the data acquisition scheme, and determines whether the device was experiencing motion at any of the time points. Since motion during data capture will prevent calibration, a time point is discarded if motion was detected. According to an embodiment, a new data acquisition scheme may be scheduled, or the existing data may be utilized after one or more time points are discarded. The motion could be based on gyroscope data, but could also utilize other sensors such as an accelerometer or other indicators such as an active motor.

At step 450 of the method, the oral care device determines a calibration model using the obtained sensor data. According to an embodiment, the system selects a calibration model based on one or more of: (i) data obtained during the scheduled acquisition; (ii) prior knowledge about the expected model; and (iii) a requested accuracy, which can be determined by a user or preprogrammed or predetermined by manufacturer settings. According to an embodiment, the complexity of the model can be adapted to the available data. For example, initially only a calibration offset will be estimated, although when sufficient data is present over a sufficiently large temperature range, a higher order polynomial calibration model can be estimated.

At step 460 of the method, the oral care device generates, based on the obtained sensor data and the determined calibration model, one or more parameters describing a behavior of the gyroscope. Commonly used techniques like least squares estimates can be utilized for the generation of parameters. According to an embodiment, the one or more parameters are generated before each cleaning session, assuming that new calibration data is available. However, if there is currently a valid calibration model, the parameters can be generated during and/or after use as well.

At step 470 of the method, the oral care device calibrates gyroscope data obtained during a cleaning session using the generated one or more parameters and the selected model. According to an embodiment, the system uses the selected model and one or more parameters to correct for the offset in the measured gyroscope signals.

At optional step 480 of the method, the oral care device tests the validity or accuracy of the determined calibration model. For example, according to an embodiment, the validity of the select model can be determined at a given condition, such as a known temperature, using the determined calibration model, the one or more parameters, and the stored data points. If the determined calibration model is inaccurate, perhaps determined by a threshold inaccuracy or other method, then user intervention may be necessary to calibrate the gyroscope. Accordingly, at optional step 490 of the method, the device requests that the user perform a calibration. This can be communicated to the user via a user interface, a visual, audible, or haptic cue, through a smartphone app, or via any other mechanism. The calibration can be to lay the device on a flat surface, place the device in a warm or cold room, or other step or mechanism. According to an embodiment, the system automatically determines that the user has complied with the request by receiving data from the one or more sensors 28 indicating that the device is in the proper orientation and/or is not experiencing motion.

## Claims

1. A method (400) for calibrating a gyroscope (28) of an oral care device (10), the method comprising the steps of:
scheduling (420) a data acquisition scheme comprising a plurality of time points at two or more different temperatures;
obtaining (430) sensor data by the gyroscope at each of the plurality of time points;
determining (440) whether the oral care device was experiencing motion at any of the plurality of time points and, if so, discarding those time points from further analysis;
determining (450) a calibration model using the obtained sensor data;
generating (460), based on the obtained sensor data and the determined calibration model, one or more parameters of the calibration model; and
calibrating (470), using the calibration model and the one or more parameters, gyroscope data obtained during a cleaning session.

2. The method of claim 1, wherein the calibration model comprises a calibration offset.

3. The method of claim 1, wherein the data acquisition scheme is based at least in part on observed temperature data.

4. The method of claim 1, wherein the data acquisition scheme is based at least in part on one or more expected temperatures.

5. The method of claim 1, wherein the data acquisition scheme is designed to maximize a range of temperatures for the plurality of time points, and minimize a chance of physical motion at the plurality of time points.

6. The method of claim 1, further comprising the step of testing (480) the determined calibration model at a known temperature, using the obtained sensor data, the determined calibration model and the one or more parameters.

7. The method of claim 6, further comprising the step of requesting (490), if the determined calibration model is inaccurate, a calibration by a user of the oral care device.

8. An oral care device (10) configured to calibrate a gyroscope (28) of the oral care device, the device comprising:
a temperature module configured to provide temperature data; and
a controller (30) configured to:
(i) schedule a data acquisition scheme comprising a plurality of time points at two or more different temperatures;
(ii) receive sensor data from the gyroscope for each of the plurality of time points;
(iii) determine whether the oral care device was experiencing motion at any of the plurality of time points and, if so, discarding those time points from further analysis;
(iv) determine a calibration model using the obtained sensor data;
(v) generate, based on the obtained sensor data and the determined calibration model, one or more parameters of the calibration model; and
(vi) calibrate, using the calibration model and the one or more parameters, gyroscope data obtained during a cleaning session.

9. The oral care device of claim 8, further comprising the gyroscope (28) configured to obtain gyroscope data.

10. The oral care device of claim 8, wherein the calibration model comprises a calibration offset.

11. The oral care device of claim 8, wherein the data acquisition scheme is based at least in part on observed temperature data.

12. The oral care device of claim 8, wherein the controller is further configured to test the determined calibration model at a known temperature, using the obtained sensor data, the determined calibration model and the one or more parameters.

13. The oral care device of claim 12, wherein the controller is further configured to request, if the determined calibration model is inaccurate, a calibration by a user of the oral care device.

## Patentansprüche

1. Verfahren (400) zum Kalibrieren eines Gyroskops (28) einer Mundpflegevorrichtung (10), wobei das Verfahren die folgenden Schritte umfasst:
Planen (420) eines Datenerfassungsschemas, das mehrere Zeitpunkte bei zwei oder mehr verschiedenen Temperaturen umfasst;
Erhalten von (430) Sensordaten durch das Gyroskop an jedem der mehreren Zeitpunkte;
Bestimmen (440), ob die Mundpflegevorrichtung an einem der mehreren Zeitpunkte eine Bewegung erfahren hat, und, falls dies der Fall ist, diese Zeitpunkte aus der weiteren Analyse zu streichen;
Bestimmen (450) eines Kalibrierungsmodells unter Verwendung der erhaltenen Sensordaten;
Erzeugen (460) eines oder mehrerer Parameter des Kalibrierungsmodells basierend auf den erhaltenen Sensordaten und dem bestimmten Kalibrierungsmodell; und
Kalibrieren (470) von Gyroskopdaten, die während einer Reinigungssitzung erhalten wurden, unter Verwendung des Kalibrierungsmodells und eines oder mehrerer Parameter.

2. Verfahren nach Anspruch 1, wobei das Kalibrierungsmodell einen Kalibrierungsversatz umfasst.

3. Verfahren nach Anspruch 1, wobei das Datenerfassungsschema zumindest teilweise auf beobachteten Temperaturdaten basiert.

4. Verfahren nach Anspruch 1, wobei das Datenerfassungsschema zumindest teilweise auf einer oder mehreren erwarteten Temperaturen basiert.

5. Verfahren nach Anspruch 1, wobei das Datenerfassungsschema ausgelegt ist, um einen Temperaturbereich für mehrere Zeitpunkte zu maximieren und eine Wahrscheinlichkeit einer physischen Bewegung an mehreren Zeitpunkten zu minimieren.

6. Verfahren nach Anspruch 1, ferner umfassend den Schritt des Testens (480) des bestimmten Kalibrierungsmodells bei einer bekannten Temperatur unter Verwendung der erhaltenen Sensordaten, des bestimmten Kalibrierungsmodells und des einen oder der mehreren Parameter.

7. Verfahren nach Anspruch 6, ferner umfassend den Schritt des Anforderns (490) einer Kalibrierung durch einen Benutzer des Mundpflegegeräts, wenn das bestimmte Kalibrierungsmodell ungenau ist.

8. Mundpflegevorrichtung (10), konfiguriert zum Kalibrieren eines Gyroskops (28) der Mundpflegevorrichtung, wobei die Vorrichtung umfasst:
ein Temperaturmodul, das konfiguriert ist, um Temperaturdaten bereitzustellen; und
eine Steuerung (30), die konfiguriert ist, zum:
(i) Planen eines Datenerfassungsschemas, das mehrere Zeitpunkte bei zwei oder mehr verschiedenen Temperaturen umfasst;
(ii) Empfangen von Sensordaten vom Gyroskop für jeden der mehreren Zeitpunkte;
(iii) Bestimmen, ob das Mundpflegegerät an einem der mehreren Zeitpunkte eine Bewegung erfahren hat, und, falls dies der Fall ist, diese Zeitpunkte aus der weiteren Analyse zu streichen;
(iv) Bestimmen eines Kalibrierungsmodells unter Verwendung der erhaltenen Sensordaten;
(v) Erzeugen eines oder mehrerer Parameter des Kalibrierungsmodells basierend auf den erhaltenen Sensordaten und dem bestimmten Kalibrierungsmodell; und
(vi) Kalibrieren von Gyroskopdaten, die während einer Reinigungssitzung erhalten wurden, unter Verwendung des Kalibrierungsmodells und des einen oder der mehreren Parameter.

9. Mundpflegevorrichtung nach Anspruch 8, ferner umfassend das Gyroskop (28), das konfiguriert ist, um Gyroskopdaten zu erhalten.

10. Mundpflegevorrichtung nach Anspruch 8, wobei das Kalibrierungsmodell einen Kalibrierungsversatz umfasst.

11. Mundpflegevorrichtung nach Anspruch 8, wobei das Datenerfassungsschema zumindest teilweise auf beobachteten Temperaturdaten basiert.

12. Mundpflegevorrichtung nach Anspruch 8, wobei die Steuerung ferner konfiguriert ist, um das bestimmte Kalibrierungsmodell bei einer bekannten Temperatur unter Verwendung der erhaltenen Sensordaten, des bestimmten Kalibrierungsmodells und des einen oder der mehreren Parameter zu testen.

13. Mundpflegevorrichtung nach Anspruch 12, wobei die Steuerung ferner konfiguriert ist, um, wenn das bestimmte Kalibrierungsmodell ungenau ist, eine Kalibrierung durch einen Benutzer der Mundpflegevorrichtung anzufordern.

## Revendications

1. Procédé (400) pour étalonner un gyroscope (28) d'un dispositif de soins buccaux (10), le procédé comprenant les étapes consistant à:
planifier (420) un schéma d'acquisition de données comprenant une pluralité de points temporels à deux ou plusieurs températures différentes;
obtenir (430) des données de capteur par le gyroscope en chacun de la pluralité de points temporels;
déterminer (440) si le dispositif de soins buccaux a subi un mouvement à niveau de l'un quelconque de la pluralité de points temporels et, si tel est le cas, éliminer ces points temporels d'une analyse ultérieure;
déterminer (450) un modèle d'étalonnage en utilisant les données de capteur obtenues;
générer (460), sur la base des données de capteur obtenues et du modèle d'étalonnage déterminé, un ou plusieurs paramètres du modèle d'étalonnage; et
étalonner (470), en utilisant le modèle d'étalonnage et l'un ou les plusieurs paramètres, des données de gyroscope obtenues pendant une session de nettoyage.

2. Procédé selon la revendication 1, dans lequel le modèle d'étalonnage comprend un décalage d'étalonnage.

3. Procédé selon la revendication 1, dans lequel le schéma d'acquisition de données est basé au moins en partie sur des données de température observées.

4. Procédé selon la revendication 1, dans lequel le schéma d'acquisition de données est basé au moins en partie sur une ou plusieurs températures attendues.

5. Procédé selon la revendication 1, dans lequel le schéma d'acquisition de données est conçu pour maximiser une plage de températures pour la pluralité de points temporels, et minimiser un risque de mouvement physique à niveau de la pluralité de points temporels.

6. Procédé selon la revendication 1, comprenant en outre l'étape de test (480) du modèle d'étalonnage déterminé à une température connue, en utilisant les données de capteur obtenues, le modèle d'étalonnage déterminé et l'un ou les plusieurs paramètres.

7. Procédé selon la revendication 6, comprenant en outre l'étape de demande (490), si le modèle d'étalonnage déterminé est inexact, d'un étalonnage par un utilisateur du dispositif de soins buccaux.

8. Dispositif de soins buccaux (10) configuré pour étalonner un gyroscope (28) du dispositif de soins buccaux, le dispositif comprenant:
un module de température configuré pour fournir des données de température; et
un dispositif de commande (30) configuré pour:
(i) planifier un schéma d'acquisition de données comprenant une pluralité de points temporels à deux ou plusieurs températures différentes;
(ii) recevoir des données de capteur du gyroscope pour chacun de la pluralité de points temporels;
(iii) déterminer si le dispositif de soins buccaux a subi un mouvement à niveau de l'un quelconque de la pluralité de points temporels et, si tel est le cas, éliminer ces points temporels d'une analyse ultérieure;
(iv) déterminer un modèle d'étalonnage en utilisant les données de capteur obtenues;
(v) générer, sur la base des données de capteur obtenues et du modèle d'étalonnage déterminé, un ou plusieurs paramètres du modèle d'étalonnage; et
(vi) étalonner, en utilisant le modèle d'étalonnage et l'un ou les plusieurs paramètres, des données de gyroscope obtenues pendant une session de nettoyage.

9. Dispositif de soins buccaux selon la revendication 8, comprenant en outre le gyroscope (28) configuré pour obtenir des données de gyroscope.

10. Dispositif de soins buccaux selon la revendication 8, dans lequel le modèle d'étalonnage comprend un décalage d'étalonnage.

11. Dispositif de soins buccaux selon la revendication 8, dans lequel le schéma d'acquisition de données est basé au moins en partie sur des données de température observées.

12. Dispositif de soins buccaux selon la revendication 8, dans lequel le dispositif de commande est en outre configuré pour tester le modèle d'étalonnage déterminé à une température connue, en utilisant les données de capteur obtenues, le modèle d'étalonnage déterminé et l'un ou les plusieurs paramètres.

13. Dispositif de soins buccaux selon la revendication 12, dans lequel le dispositif de commande est en outre configuré pour demander, si le modèle d'étalonnage déterminé est inexact, un étalonnage par un utilisateur du dispositif de soins buccaux.
